# EUROPEAN PATENT SPECIFICATION

(11) **EP 4 161 452 B1**
(45) Date of publication and mention of the grant of the patent: **25.12.2024**
(21) Application number: 21736367.0
(22) Date of filing: 01.06.2021
(51) Int. Cl.: A61F 2/66, A61F 2/50

(54) **PROSTHETIC ANKLE-FOOT DEVICE, IN PARTICULAR OF THE BIOMIMETIC TYPE**
KNÖCHEL-FUSS-PROTHESE, INSBESONDERE VOM BIOMIMETISCHEN TYP
DISPOSITIF PROTHÉTIQUE DE CHEVILLE-PIED, EN PARTICULIER DE TYPE BIOMIMÉTIQUE

(30) Priority: 03.06.2020 IT 202000013141
(43) Date of publication of application: 12.04.2023
(73) Proprietor: Università Degli Studi Di Padova, 35122 Padova (IT)
(72) Inventor: PETRONE, Nicola, 35027 Noventa Padovana (PD) (IT); MISTRETTA, Paolo, 35121 Padova (PD) (IT); FAGGIAN, Riccardo, 30031 Dolo (PD) (IT)
(74) Representative: Metroconsult Srl
(86) International application number: PCT/IB2021/054798
(87) International publication number: WO 2021/245552

(56) References cited:
- WO-A1-2018/166905
- WO-A1-2019/045146
- DE-A1- 10 010 302
- DE-A1- 102005 062 231
- SU-A1- 848 023
- US-A1- 2020 085 596

## Description

The present invention relates to a prosthetic ankle-foot device, in particular of the biomimetic type, according to the preamble of claim 1.

Several types of prosthetic ankle-foot devices are known in the art, which can be divided into three big categories: conventional feet (CF), energy storing and returning feet (ESR), and bionic feet (BIO).

Conventional feet, which were developed first, have no concentrated-type degree of freedom, and cannot therefore make physiological movements; their main function is to damp the forces generated by the impact of the foot on the ground and transfer them to the tibia.

Energy storing and returning feet may be either of the single-joint type, typically having one degree of freedom corresponding to the ankle (talocrural) joint, or of the multijoint type, wherein the prosthesis can move about different articulation axes, more or less corresponding to physiological movements. Bionic feet can be divided into two types, i.e. stabilizing (also referred to as "quasi-active") bionic feet and propulsive (also referred to as "active") bionic feet. In the former, the active components (usually consisting of motors) perform the function of adjusting some of the prosthesis' features, e.g. ankle rigidity or foot angle, in order to adapt to different sloping grounds. In the latter, the active components perform the function of supplying power to the joints (in particular, the talocrural one), thus causing the prosthesis to move according to a given control logic.

At present, most commercial prosthetic devices fall within the energy storing and returning feet (ESR) category, since only a few stabilizing bionic prostheses and just one propulsive bionic prosthesis is currently available on the market. Typical commercial prostheses include a main element corresponding to the ankle-foot assembly and made of deformable material, e.g. carbon-fiber sheet, which allows relative movement between the foot and the leg due to the deformability of the material.

Most prostheses currently undergoing research and development have just one degree of freedom, typically associated with the ankle joint (i.e. the talocrural joint), since the foot is usually considered as a secondary, non-functional element.

It is therefore apparent that such prosthetic devices do not permit the achievement of a mechanical behaviour of the prosthesis that is comparable to that of the biologic ankle-foot system, which has been optimized by genetic evolution and therefore represents an optimal trade-off between different functionalities such as, for example, adaptability to different grounds (thanks to the large number of joints), propulsive capability (due to the presence of muscles), and energetic efficiency (due to the presence of elastic elements capable of storing and releasing energy).

It must also be pointed out that people who have suffered amputation and wearers of lower-limb prosthetic devices establish compensation mechanisms which result in gait asymmetry and which may lead to the onset of secondary disorders (osteoarthritis, osteoporosis, and the like) and/or to higher metabolic energy consumption, inevitably resulting in increased fatigue.

US 2020/085596 discloses a foot prosthesis comprising a heel and a foot tip capable of bearing on the ground and an ankle support wherein it further comprises at least one damping element configured to be distant from said ground.

US10,292,840B2 relates to a passive prosthetic device made up of several parts. In fact, the device described in document US10,292,840B2 comprises a phalanx portion, a metatarsal portion that is movably coupled to the phalanx portion, an ankle portion that is movably connected to the phalanx portion, and a calcaneus portion that is movably coupled to the ankle portion, wherein each coupling between the parts of the device is effected by means of a torsion spring.

Although in document US10,292,840B2 the foot is considered as a functional element, also the prosthetic device described in such document suffers from a few drawbacks.

In particular, such drawbacks are due to the fact that the phalanx portion and the metatarsal portion made in accordance with the teachings of document US10,292,840B2 consist of solid, rigid bodies that do not allow the prosthetic device to achieve adequate adaptability to different grounds, especially when such grounds are rough and/or unstable.

Moreover, the coupling of the parts of the device by means of torsion springs does not ensure an adequate mechanical connection between the forefoot joint and the plantar arch, which is a peculiar characteristic of the biologic ankle-foot complex.

In this frame, it is the main object of the present invention to provide a prosthetic ankle-foot device, in particular of the biomimetic type, which is so conceived as to overcome the drawbacks of the prior art.

In particular, it is one object of the present invention to provide a prosthetic ankle-foot device so realized as to permit the achievement of a mechanical behaviour which is comparable to that of the biologic ankle-foot system.

It is another object of the present invention to provide a prosthetic ankle-foot device which offers adequate adaptability to different grounds and high energetic efficiency, being realized to provide optimal energy storage and release.

It is a further object of the present invention to provide a prosthetic ankle-foot device so designed as to minimize the establishment of compensation mechanisms by a person wearing said device and to avoid any gait asymmetry, thus preventing the onset of secondary disorders and/ or a higher consumption of metabolic energy which would result in increased fatigue.

It is another object of the present invention to provide a prosthetic ankle-foot device so realized as to make it possible to assess the effect of a single factor or parameter on a specific functionality of the prosthetic device.

It is yet another object of the present invention to provide a prosthetic ankle-foot device that is not too expensive to manufacture and difficult to set up.

Further objects, features and advantages of the present invention will become apparent in the light of the following detailed description and the annexed drawings, which are supplied by way of non-limiting explanatory example, wherein:
- Fig. 1 is a perspective view of a prosthetic ankle-foot device, in particular of the biomimetic type, according to the present invention;
- Fig. 2 is an exploded perspective view of the prosthetic device according to the present invention shown in Fig. 1;

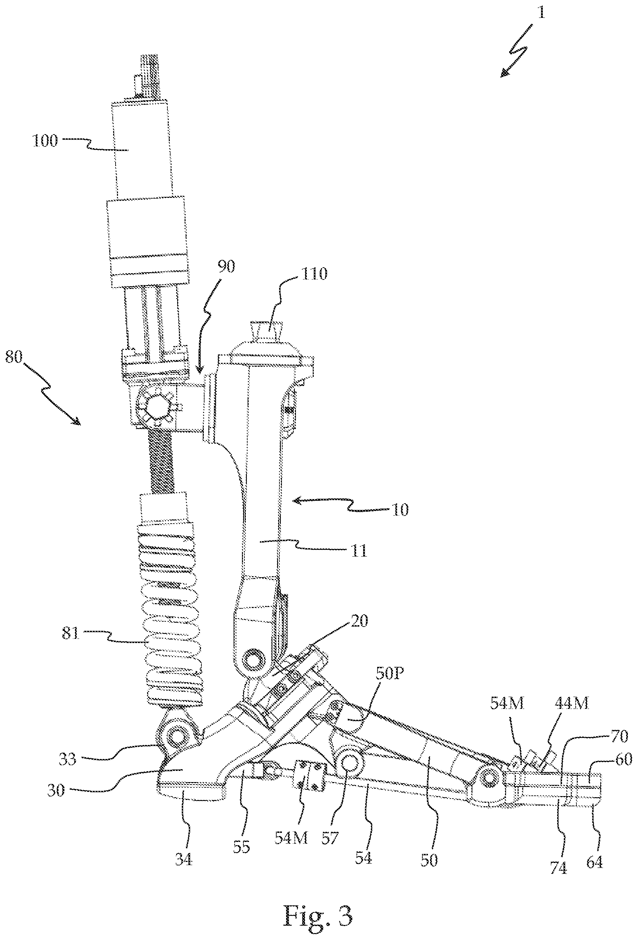

- Fig. 3 is a lateral view of the prosthetic device according to the present invention;
- Fig. 4 is an exploded lateral view of the prosthetic device shown in Fig. 3.

Referring now to the annexed drawings, reference numeral 1 designates as a whole a prosthetic ankle-foot device, in particular of the biomimetic type, according to the present invention.

In accordance with the present invention, the device 1 comprises a tibia element 10, a talus element 20 movably connected to the tibia element 10 through a first talocrural joint A1 comprising a first hinge joint, and a calcaneus element 30 movably connected to the talus element 20 through a second subtalar joint A2 comprising a second hinge joint.

In addition, the device 1 comprises a medial metatarsal element 40 movably connected to the calcaneus element 30 through a third medial midtarsal joint A3, said third joint A3 comprising a third hinge joint, and a lateral metatarsal element 50 movably connected to the calcaneus element 30 through a fourth lateral midtarsal joint A4, said fourth joint A4 comprising a fourth hinge joint. The device 1 according to the present invention further comprises a medial phalanx element 60 movably connected to the medial metatarsal element 40 through a fifth medial metatarsophalangeal joint A5, said fifth joint A5 comprising a fifth hinge joint, and a lateral phalanx element 70 movably connected to the lateral metatarsal element 50 through a sixth lateral metatarsophalangeal joint A6, said sixth joint A6 comprising a sixth hinge joint. It should be noted that said hinge joints are preferably implemented as respective hinges, in particular cylindrical hinges.

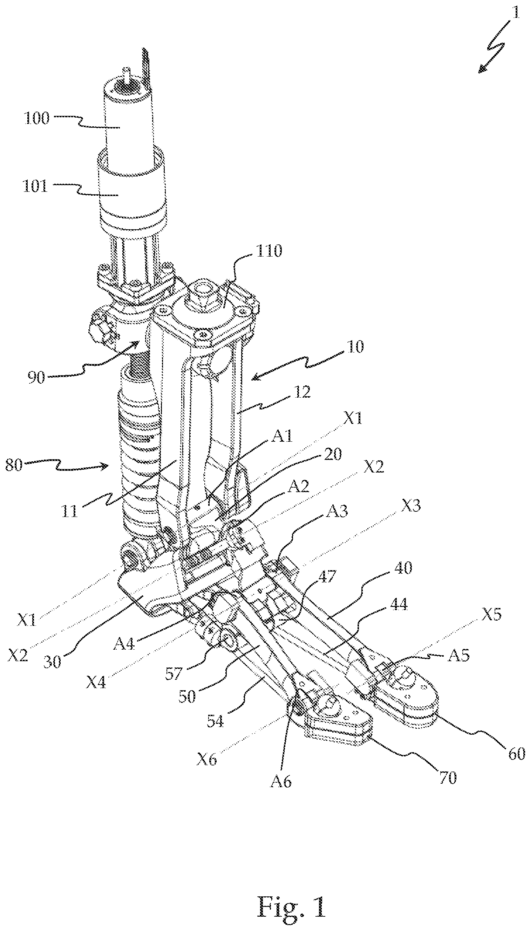

The device 1 according to the present invention further comprises an elastic actuation element (designated as a whole by reference numeral 80 in the annexed drawings) comprising an upper part movably connected to the tibia element 10 and a lower part movably connected to the calcaneus element 30. In a preferred embodiment, the tibia element 10 has a fork-like shape and comprises a first arm 11 and a second arm 12 substantially parallel to each other. In accordance with the present invention, the first talocrural joint A1 lies on a first axis X1 and the second subtalar joint A2 lies on a second axis X2, wherein said first axis X1 and second axis X2 (shown in Fig. 1) form an oblique ankle dual axis, in particular having biomimetic orientation and position; as a consequence, said oblique dual axis turns out to be similar to the biologic ankle complex, i.e. similar to the physiological talocrural joint and subtalar joint.

In particular, the talocrural axis is inclined relative to the midlateral axis by approximately 10° in the frontal (or coronal) anatomical plane and by approximately 6° in the horizontal (or transversal) plane; as to the subtalar axis, it is inclined relative to the sagittal axis by approximately 42° in the sagittal plane and by approximately 16° in the horizontal plane.

Like its biological counterpart, the first talocrural joint A1 is responsible for the physiological dorsiflexion and plantarflexion movements, i.e. the flexion and extension movements of the ankle; the rigidity of the first talocrural joint A1 is given by the presence of the actuation element 80, and the range of movement of said first talocrural joint A1 is such as to allow different gait types according to a physiological biomechanical pattern, including, without being limited to, walking on flat ground, walking uphill and downhill, and climbing and descending stairs.

In a preferred embodiment, the first talocrural joint A1 comprises a first aperture 11A associated with the first arm 11 of the tibia element 10 and a second aperture 12A associated with the second arm 12 of the tibia element 10, wherein said apertures 11A, 12A are coupled to a talocrural pin 21 integral with the talus element 20; preferably, each one of said apertures 11B, 12B comprises a bushing, in particular of the self-lubricating type.

As far as the second subtalar joint A2 is concerned, it is responsible, just like the corresponding biological joint, for pronation and supination movements.

The second subtalar joint A2 comprises a hole 22 on the talus element 20, which is coupled to a subtalar pin 31 integral with the calcaneus element 30; preferably, said hole 22 houses at least one bushing, in particular of the self-lubricating type.

Moreover, the second subtalar joint A2 is so realized as to have a rigidity of its own. For this purpose, the second subtalar joint A2 comprises a subtalar pad 23 positioned between opposed faces of the talus element 20 and of the calcaneus element 30; preferably, said subtalar pad 23 is made of elastomeric material, and in such a way as to obtain a rigidity of the second subtalar joint A2 that substantially corresponds to that of the biological counterpart.

The particular construction of the subtalar pad 23 makes it possible to specifically design the rigidity curve of the second subtalar joint A2 as needed, even as a non-linear one.

The third medial midtarsal joint A3 and the fourth lateral midtarsal joint A4 are substantially parallel and coaxial to each other, said joints A3, A4 lying on a third axis X3 and a fourth axis X4, respectively, which substantially coincide; for this reason, the third axis X3 and the fourth axis X4 are represented in Fig. 1 as a single dashed-dotted line.

According to a preferred embodiment, the third medial midtarsal joint A3 comprises a first pin 32A integral with the calcaneus element 30, which articulates with a hole 41 that is present on a proximal part of the medial metatarsal element 40, in particular said hole 41 being associated with at least one radial bearing or a self-lubricating bushing.

Furthermore, the fourth lateral midtarsal joint A4 comprises a second pin 32B integral with the calcaneus element 30, which articulates with a hole 51 that is present on a proximal part of the lateral metatarsal element 50, in particular said hole 51 being associated with at least one self-lubricating bushing.

The fifth medial metatarsophalangeal joint A5 and the sixth lateral metatarsophalangeal joint A6 lie on a fifth axis X5 and a sixth axis X6, respectively, which are substantially parallel and not coinciding.

The fifth medial metatarsophalangeal joint A5 consists of a hinge joint comprising a medial metatarsophalangeal pin 42 which articulates with at least one first aperture 43 that is present on a distal part of the medial metatarsal element 40 and with a second aperture 62 that is present on an arm 61 extending from the medial phalanx element 60.

In its turn, the sixth lateral metatarsophalangeal joint A6 consists of a hinge joint comprising a lateral metatarsophalangeal pin 52 which articulates with at least one first aperture 53 that is present on a distal part of the lateral metatarsal element 50 and with a second aperture 72 that is present on an arm 71 extending from the lateral phalanx element 70.

It is therefore clear that the tibia element 10, the talus element 20 and the calcaneus element 30 find a direct biological counterpart in the biological tibia, talus and calcaneus, respectively. As concerns the medial metatarsal element 40, the lateral metatarsal element 50, the medial phalanx element 60 and the lateral phalanx element 70, they do not have a direct biological counterpart, since they are "functional" elements. In fact, the medial metatarsal element 40 mimics the function of the first and second biological metatarsi, while the lateral metatarsal element 50 mimics the third, fourth and fifth biological metatarsi; the same also applies to the medial phalanx element 60 and the lateral phalanx element 70. This division into functional groups allows reducing the complexity of the device 1 according to the present invention, while at the same time maintaining the main functionalities of the corresponding biological elements. In particular, the structure of the device 1 according to the present invention makes it possible to obtain three plantar arches (medial longitudinal, lateral longitudinal and transverse), while at the same time permitting the coupling between the plantar arches (midtarsal joints) and the metatarsophalangeal joints.

In this frame, the first talocrural joint A1 and the second subtalar joint A2 constitute the joints of an ankle complex of the device 1 according to the present invention, and have a direct biological counterpart, in that they mimic its position and spatial orientation.

The remaining four joints (i.e. the third medial midtarsal joint A3, the fourth lateral midtarsal joint A4, the fifth medial metatarsophalangeal joint A5 and the sixth lateral metatarsophalangeal joint A6) constitute the joints of the foot of the device 1 according to the present invention and do not have a direct biological counterpart, in that they are functional joints. In this frame, the two midtarsal joints A3, A4 mimic the function of the biological midtarsal joint (also referred to as Chopart's joint), which is composed of the calcaneocuboid and talonavicular joints, while the two metatarsophalangeal joints A5, A6 mimic the biological function of the five biological metatarsophalangeal joints. Moreover, said four joints A3, A4, A5, A6 constitute a single functional group, just like in the biological foot, where there is a direct coupling between midtarsal and metatarsophalangeal joints. In particular, the complex formed by the calcaneus element 30, the third medial midtarsal joint A3, the medial metatarsal element 40, the fifth medial metatarsophalangeal joint A5 and the medial phalanx element 60 constitutes the medial longitudinal arch. Likewise, the complex formed by the calcaneus element 30, the fourth lateral midtarsal joint A4, the lateral metatarsal element 50, the sixth lateral metatarsophalangeal joint A6 and the lateral phalanx element 70 constitutes the lateral longitudinal arch. In addition, the transverse (or transversal, or anterior) arch is formed as a consequence of the presence of the medial longitudinal arch and the lateral longitudinal arch between the heads of the medial metatarsal element 40 and of the lateral metatarsal element 50.

As can be seen in the annexed drawings, the actuation element 80 preferably comprises a spring 81, in particular a coil spring, connected to an upper body 82 which articulates with the tibia element 10 through a pivotable fork system allowing two degrees of freedom between said actuation element 80 and the tibia element 10.

In this respect, the pivotable fork system according to the present invention comprises a fork-shaped element 90 and first connection elements which allow a first relative rotational motion between the actuation element 80 and the fork-shaped element 90; note that said first rotational motion constitutes the first degree of freedom between the actuation element 80 and the tibia element 10. In the embodiment shown in the annexed drawings, said first connection elements comprise radial bearings 82C associated with the upper body 82 of the actuation element 80, which articulates with respective pins 91P associated with the arms 91 of the fork-shaped element 90.

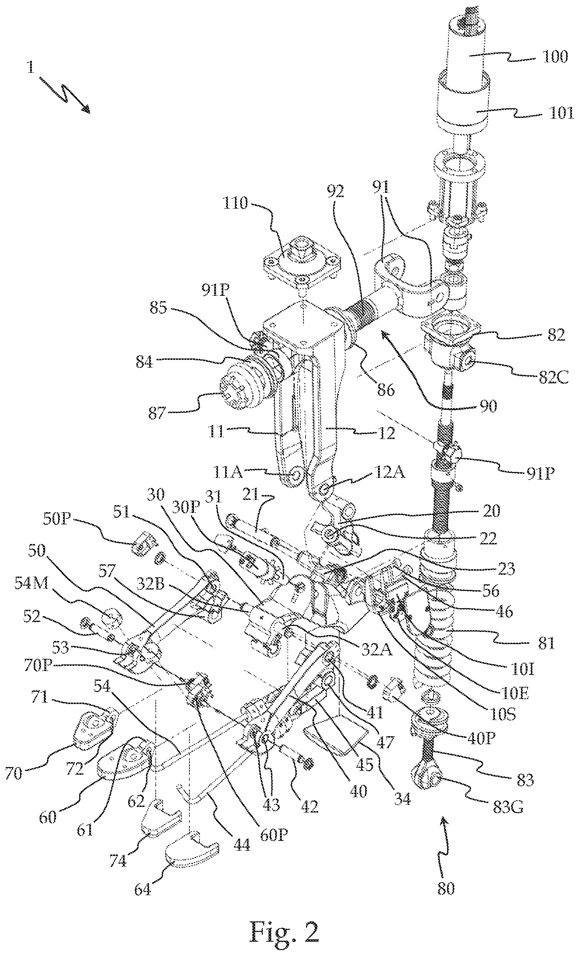

Furthermore, the pivotable fork system according to the present invention comprises second connection elements (designated by reference numerals 84, 85, 86 and 87 in Figure 2), which allow a second relative rotational motion between the actuation element 80 and the fork-shaped element 90; note that said second rotational motion constitutes the second degree of freedom between the actuation element 80 and the tibia element 10. Preferably, said second connection elements comprise at least one bearing 84, 85, 86 mounted to a rod 92 of the fork-shaped element 90, which articulates with at least one respective seat (not shown in the annexed drawings) associated with the tibia element 10. In the embodiment shown in Fig. 2, one can see that said second connection elements comprise a plurality of bearings, i.e.:
- a first axial needle bearing 84 acting axially between a circular crown that is present in the tibia element 10 and a corresponding area that is present on the castellated nut 87;
- a radial needle bearing 85 acting radially in a hole of the tibia element 10 in which the rod 92 of the fork-shaped element 90 is housed;
- a second axial needle bearing 86 acting axially between the circular crown that is present in the tibia element 10 and a front face of the fork-shaped element 90.

Said second connection elements comprise also a locking element 87 which is coupled to the rod 92 of the fork-shaped element 90 to tighten the bearings 84, 85, 86 against the tibia element 10; when observing Fig. 2, it can be noticed that said locking element 87 may consist of a castellated nut, possibly associated with a cotter pin (not shown) to prevent it from turning about said rod 92. The lower part of the actuation element 80 articulates with the calcaneus element 30 through a terminal 83 that allows two degrees of freedom between said actuation element 80 and calcaneus element 30. In the embodiment shown in the annexed drawings, said terminal 83 articulates with a fork 33 of the calcaneus element 30 and comprises a ball joint 83G associated with anti-torsion elements; in particular, said anti-torsion elements comprise lateral faces of the ball joint 83G provided with elements tangent to the inner faces of said fork 33. Such an embodiment permits locking exclusively an undesired third degree of freedom of the ball joint 83G, i.e. the degree of freedom corresponding to torsion of the actuation element 80.

In accordance with a preferred embodiment, the device 1 comprises a motor 100, in particular of the electric type, associated with the upper body 82 of said actuation element 80, possibly through the interposition of a reducer 101.

It is clear that, due to the provision of the motor 100, the prosthetic device 1 of the present invention becomes active, since it can produce net positive power. In this context, the assembly made up of the actuation element 80 and the motor 100 reproduces the positioning (i.e. the origin and the insertion) of the biological soleus muscle, which is a biarticular muscle because it acts simultaneously upon both the talocrural joint and the subtalar joint. It should be noted that the particular implementation shown in the annexed drawings permits minimizing the peak power required from the motor 100 in order to cause the device 1 to make the walking gesture.

Furthermore, the assembly made up of the actuation element 80 and the motor 100 reproduces the functionalities of the whole set of biological plantarflexor and dorsiflexor muscles; however, unlike biological muscles, this assembly can operate in a desmodromic manner both when pulling (plantarflexion) and when pushing (dorsiflexion), and can produce all the power of the complex of plantarflexor (mainly soleus, medial and lateral gastrocnemius) and dorsiflexor (mainly anterior tibial) muscles.

It should be noted that, in accordance with the teachings of the present invention, the actuation element 80 is positioned in series with the motor 100, just like the tendon is in series with the active element (muscle) in the biological counterpart.

In accordance with a preferred embodiment, the rigidity of the actuation element 80, in particular the spring 81, must be such as to provide a trade-off among: minimization of the peak power of the electric motor during the walking cycle, minimization of the energy consumed by the electric motor during the walking cycle, and possibility of using the prosthesis with a locked motor (motor turned off). It has been observed, in fact, that the optimal rigidity of the active actuation system including the motor 100 (minimization of peak power and energy consumption) corresponds, with due approximation, to the optimal rigidity that would be necessary if the device 1 did not include the motor 100 and were, as a consequence, totally passive (linear regression of the torque vs. ankle angle curve). Note that such optimal rigidity also corresponds, with due approximation, to the physiological rigidity of the Achilles tendon (tendon of the plantarflexor muscles).

The prosthetic device 1 according to the present invention can therefore be made to operate correctly also in "passive mode", by preventing the rotation of the electric motor 100 or by building the device 1 without associating the motor 100 with the actuation element 80.

From the above description it clearly emerges that the kinematic mechanism formed by the tibia element 10, the talus element 20, the calcaneus element 30, the actuation element 80 and the fork-shaped element 90, possibly also including the motor 100, constitutes a closed-chain spatial kinematic mechanism, as opposed to a planar kinematic mechanism like those of prior-art devices.

In this context, the pivotable fork system with two degrees of freedom that connects the upper part of the actuation element 80 to the tibia element 10 and the ball joint reduced to a double cylindrical joint that connects the lower part of the actuation element 80 to the calcaneus element 30 perform a dual function. Firstly, such an embodiment ensures a smooth actuation of a system with two degrees of freedom, made up of the first talocrural joint A1 and the second subtalar joint A2 coupled together; it is thus possible to actuate said system with two degrees of freedom between the tibia element 10 and the calcaneus element 30 (as in the biological ankle-foot complex), instead of actuating one degree of freedom at a time with planar mechanisms. Secondly, such an embodiment makes it possible (even though if the mechanism is a spatial kinematic mechanism) to obtain the previously described desmodromic system, i.e. a system capable of both pulling and pushing.

In accordance with the present invention, the device 1 comprises:
- a first elastic element 44, which connects the calcaneus element 30 to the medial phalanx element 60 and which is positioned under the medial metatarsal element 40,
- a second elastic element 54, which connects the calcaneus element 30 to the lateral phalanx element 70 and which is positioned under the lateral metatarsal element 50.

The connection between the elastic elements 44, 54 and the calcaneus element 30, the medial phalanx element 60 and the lateral phalanx element 70 is accomplished through suitable fastening means, e.g. respective clamps 44M, 54M.

Said elastic elements 44, 54 perform the task of reproducing the function of the biological plantar aponeurosis, in that they confer rigidity on the (medial and lateral) longitudinal plantar arches and permit the coupling between the midtarsal joints A3, A4 and the metatarsophalangeal joints A5, A6.

In a preferred embodiment, the device 1 comprises a first system 45, 46 for adjusting the tension of the first elastic element 44 and a second system 55, 56 for adjusting the tension of the second elastic element 54.

In particular, the first adjustment system comprises a first tensioner 45 connected to the first elastic element 44 and associated with a first adjustment screw 46 for causing the first tensioner 45 to slide relative to the calcaneus element 30 in order to either increase or decrease the tension of the first elastic element 44.

Furthermore, the second adjustment system comprises a second tensioner 55 connected to the second elastic element 54 and associated with a second adjustment screw 56 for causing the second tensioner 55 to slide relative to the calcaneus element 30 in order to either increase or decrease the tension of the second elastic element 54.

Said adjustment systems make it possible to calibrate and adjust the rigidity curve of the plantar arches and of the metatarsophalangeal joints A5, A6 on the basis of several factors, such as, for example, the person's weight, the mode of operation of the device 1 (walking on flat ground, walking on rough ground, etc.), or according to the user's preferences.

Preferably, the underside of the medial metatarsal element 40 comprises a first arm 47, whereon the first elastic element 44 rests, and the underside of the lateral metatarsal element 50 comprises a second arm 57, whereon the second elastic element 54 rests.

Said first arm 47 and second arm 57 have several purposes, in that they allow:
- preventing the plantar arch from collapsing, because, as the arch lowers, they will keep substantially constant the leverage ratio between the elastic elements 44, 54 and the midtarsal joints A3, A4;
- accurately setting the rigidity of the plantar arch (and hence of the metatarsophalangeal joints A5, A6) by appropriately designing the kinematic mechanism;
- leaving sufficient room for inserting elements suitable for locking the midtarsal joints A3, A4, as will be described hereinafter.

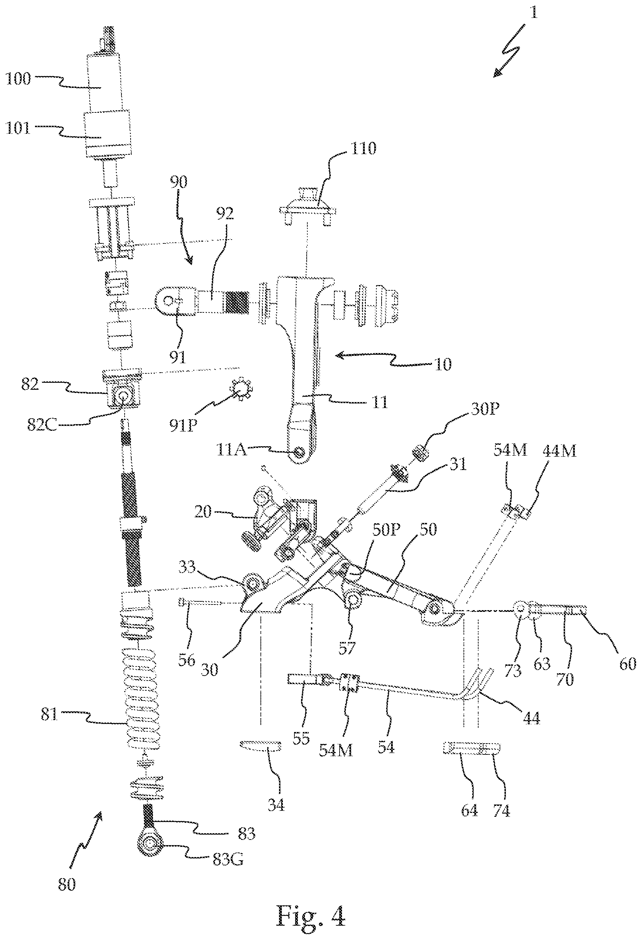

In accordance with a preferred embodiment, the medial phalanx element 60 comprises a first cam 63 (visible in Fig. 4), whereon the first elastic element 44 rests, and the lateral phalanx element 70 comprises a second cam 73 (also visible in Fig. 4), whereon the second elastic element 54 rests, wherein said first cam 63 and second cam 73 may be so realized as to accurately design the rigidity curve of the fifth medial metatarsophalangeal joint A5 and of the sixth lateral metatarsophalangeal joint A6. In substance, by changing the radius of the cams 63, 73 it is possible to modify the torsional rigidity of the respective joints A5, A6.

Preferably, the device 1 according to the present invention is so realized as to comprise at least one locking element for each one of the foot joints A3, A4, A5, A6.

In particular, the device 1 may comprise:
- a first pin (not shown in the drawings) passing through a hole in the first arm 47 and through another hole in the calcaneus element 30 for locking the third medial midtarsal joint A3;
- a second pin (not shown in the drawings) passing through a hole in the second arm 57 and through another hole in the calcaneus element 30 for locking the fourth lateral midtarsal joint A4;
- a first strap, in particular nail-shaped (not shown in the drawings), fixed between the distal part of the medial metatarsal element 40 and the medial phalanx element 60 for locking the fifth medial metatarsophalangeal joint A5;
- a second strap, in particular nail-shaped (not shown in the drawings), fixed between the distal part of the lateral metatarsal element 50 and the lateral phalanx element 70 for locking the sixth lateral metatarsophalangeal joint A6.

The possibility of selectively locking the foot joints A3, A4, A5, A6 allows for accurate and detailed quantification of the influence that each one of them has on the functions of interest of the device 1.

In accordance with a preferred embodiment, the device 1 according to the present invention is realized to comprise at least one sensor 10E, 10I, 30P, 40P, 50P, 60P, 70P associated with at least one joint A1, A2, A3, A4, A5, A6 for providing a direct reading of the data concerning such joint.

In particular, the device 1 according to the present invention is preferably so realized as to comprise one or more of the following sensors (especially visible in Fig. 2):
- an encoder 10E, in particular of the magnetic type, fastened to the tibia element 10 at the first joint A1 for reading the talocrural joint angle, in particular said encoder 10E being positioned inside a removable support 10S fastened to the tibia element 10 by fastening means;
- an inertial sensor 101 fastened to the tibia element 10 at the first talocrural joint A1 for reading the absolute tibia angles, in particular said inertial sensor 10I being housed in the same support 10S as the encoder 10E;
- a first potentiometer 30P fastened to the calcaneus element 30 at the second joint A2 for reading the subtalar joint angle;
- a second potentiometer 40P fastened to the medial metatarsal element 40 at the third joint A3 for reading the medial midtarsal angle;
- a third potentiometer 50P fastened to the lateral metatarsal element 50 at the fourth joint A4 for reading the lateral midtarsal angle;
- a fourth potentiometer 60P fastened at the fifth joint A5 for reading the medial metatarsophalangeal angle, in particular said fourth potentiometer 60P being fastened to the medial metatarsal element 40;
- a fifth potentiometer 70P fastened at the sixth joint A6 for reading the lateral metatarsophalangeal angle, in particular said fifth potentiometer 70P being fastened to the lateral metatarsal element 50.

The measurements taken by the sensors 10E, 10I, 30P, 40P, 50P, 60P, 70P permit obtaining a direct reading in real time of all six (relative) joint angles, as well as reading the absolute angles with respect to the global reference system of the tibia element 10, e.g. in terms of pitch and roll.

The data thus obtained can be used for different purposes, including the creation of robust (because based on multiple input data) control models for the motor 100, the creation of regression models for estimating all the kinematic and kinetic quantities of the prosthetic device 1 (such as ground reaction forces, center of pressure trajectory, etc.), the evaluation of the quality of the gait of the person using the prosthetic device 1, and so forth.

In accordance with a preferred embodiment, the device 1 according to the present invention comprises at least one interface element 34, 64, 74, in particular made of rubber or a similar material, for damping the ground impact forces and ensuring optimal grip on the ground.

In particular, said at least one interface element comprises one or more of the following elements:
- a first interface element 34 fixed under the calcaneus element 30;
- a second interface element 64 fixed under the medial phalanx element 60;
- a third interface element 74 fixed under the lateral phalanx element 70.

It is therefore clear that said interface elements 34, 64, 74 are similar to the fat pads found in the biological foot.

The device 1 according to the present invention has a volumetric size and a mass which are similar to those of the biological counterpart, so that it can be used by amputated people even inside normal footwear; moreover, the device 1 is easily scalable for obtaining different sizes comparable to those of the biological foot. The device 1 may be so realized as to comprise a covering, in particular made of silicone-based material, into which said device 1 can be fitted.

The device 1 according to the present invention further comprises a coupling element 110, in particular of the pyramidal type, which permits connecting the device 1 to a tibial pylon (not shown in the annexed drawings); preferably, said coupling element 110 is fixed to the top part of the tibia element 10.

The features of the prosthetic ankle-foot device 1, in particular of the biomimetic type, according to the present invention, as well as the advantages thereof, are apparent from the above description.

In fact, the device 1 according to the present invention is so realized as to permit the achievement of a mechanical behaviour which is comparable to that of the biological ankle-foot system.

Furthermore, due to the provisions of the present invention, the prosthetic ankle-foot device 1 offers good adaptability to different grounds and high energetic efficiency, being designed to provide propulsive capability as well as adequate energy storage and release.

The peculiar features of the device 1 according to the present invention make it possible to minimize the establishment of compensation mechanisms by a wearer of said device; in particular, the prosthetic ankle-foot device 1 prevents gait asymmetry, thus avoiding the onset of secondary disorders and/or a higher consumption of metabolic energy that would result in increased fatigue.

The provision of the prosthetic ankle-foot device 1 with sensors 10E, 10I, 30P, 40P, 50P, 60P, 70P makes it possible to accurately quantify the effects of single factors or parameters on a specific function of interest of the device 1.

The prosthetic ankle-foot device 1 according to the present invention is not very expensive and is easy to set up, in addition to having a volumetric size and a mass that are similar to those of its biological counterpart, which allow it to be used by amputated people also inside normal footwear; moreover, the device 1 is easily scalable for obtaining different sizes comparable to those of the biological foot.

## Claims

1. Prosthetic ankle-foot device (1), in particular of the biomimetic type, said device (1) being **characterized in that** it comprises:
- a tibia element (10);
- a talus element (20) movably connected to the tibia element (10) through a first talocrural joint (A1) comprising a first hinge joint;
- a calcaneus element (30) movably connected to the talus element (20) through a second subtalar joint (A2) comprising a second hinge joint;
- a medial metatarsal element (40) movably connected to the calcaneus element (30) through a third medial midtarsal joint (A3), said third joint (A3) comprising a third hinge joint;
**characterised by**
- a lateral metatarsal element (50) movably connected to the calcaneus element (30) through a fourth lateral midtarsal joint (A4), said fourth joint (A4) comprising a fourth hinge joint;
- a medial phalanx element (60) movably connected to the medial metatarsal element (40) through a fifth medial metatarsophalangeal joint (A5), said fifth joint (A5) comprising a fifth hinge joint;
- a lateral phalanx element (70) movably connected to the lateral metatarsal element (50) through a sixth lateral metatarsophalangeal joint (A6), said sixth joint (A6) comprising a sixth hinge joint;
- an elastic actuation element (80) comprising an upper part movably connected to the tibia element (10) and a lower part movably connected to the calcaneus element (30).

2. Device (1) according to claim 1, **characterized in that** the tibia element (10) has a fork-like shape and comprises a first arm (11) and a second arm (12) substantially parallel to each other.

3. Device (1) according to one or more of the preceding claims, **characterized in that** the first talocrural joint (A1) lies on a first axis (X1) and the second subtalar joint (A2) lies on a second axis (X2), wherein said first axis (X1) and second axis (X2) form an oblique ankle dual axis having biomimetic orientation and position, in particular the first talocrural joint (A1) comprising a first aperture (11A) associated with the first arm (11) of the tibia element (10) and a second aperture (12A) associated with the second arm (12) of the tibia element (10), wherein said apertures (11A, 12A) are coupled to a talocrural pin (21) integral with the talus element (20)..

4. Device (1) according to one or more of the preceding claims, **characterized in that** the second subtalar joint (A2) comprises a hole (22) on the talus element (20), which is coupled to a subtalar pin (31) integral with the calcaneus element (30).

5. Device (1) according to one or more of the preceding claims, **characterized in that** the second subtalar joint (A2) comprises a subtalar pad (23) positioned between opposed faces of the talus element (20) and of the calcaneus element (30), in particular said subtalar pad (23) being made of elastomeric material.

6. Device (1) according to one or more of the preceding claims, **characterized in that** the third medial midtarsal joint (A3) and the fourth lateral midtarsal joint (A4) are substantially parallel and coaxial to each other, said joints (A3, A4) lying on a third axis (X3) and a fourth axis (X4), respectively, which substantially coincide, in particular the third medial midtarsal joint (A3) comprising a first pin (32A) integral with the calcaneus element (30), which articulates with a hole (41) that is present on a proximal part of the medial metatarsal element (40), and in particular the fourth lateral midtarsal joint (A4) comprising a second pin (32B) integral with the calcaneus element (30), which articulates with a hole (51) that is present on a proximal part of the lateral metatarsal element (50).

7. Device (1) according to one or more of the preceding claims, **characterized in that** the fifth medial metatarsophalangeal joint (A5) and the sixth lateral metatarsophalangeal joint (A6) lie on a fifth axis (X5) and a sixth axis (X6), respectively, which are substantially parallel and not coinciding,
in particular the fifth medial metatarsophalangeal joint (A5) consisting of a hinge joint comprising a medial metatarsophalangeal pin (42) which articulates with at least one first aperture (43) that is present on a distal part of the medial metatarsal element (40) and with a second aperture (62) that is present on an arm (61) extending from the medial phalanx element (60),
and in particular the sixth lateral metatarsophalangeal joint (A6) consisting of a hinge joint comprising a lateral metatarsophalangeal pin (52) which articulates with at least one first aperture (53) that is present on a distal part of the lateral metatarsal element (50) and with a second aperture (72) that is present on an arm (71) extending from the lateral phalanx element (70).

8. Device (1) according to one or more of the preceding claims, **characterized in that** the actuation element (80) comprises a spring (81) connected to an upper body (82) which articulates with the tibia element (10) through a pivotable fork system allowing two degrees of freedom between said actuation element (80) and the tibia element (10), in particular said pivotable fork system comprising:
- a fork-shaped element (90),
- first connection elements which allow a first relative rotational motion between the actuation element (80) and the fork-shaped element (90),
- second connection elements (84, 85, 86, 87) which allow a second relative rotational motion between the actuation element (80) and the fork-shaped element (90),
in particular said first connection elements comprising radial bearings (82C) associated with the upper body (82) of the actuation element (80), which articulate with respective pins (91P) associated with the arms (91) of the fork-shaped element (90),
and in particular said second connection elements comprising at least one bearing (84, 85, 86) mounted to a rod (92) of the fork-shaped element (90), which articulates with at least one respective seat associated with the tibia element (10).

9. Device (1) according to one or more of the preceding claims, **characterized in that** the lower part of the actuation element (80) articulates with the calcaneus element (30) through a terminal (83) that allows two degrees of freedom between said actuation element (80) and calcaneus element (30), in particular said terminal (83) articulating with a fork (33) of the calcaneus element (30) and comprising a ball joint (83G) associated with anti-torsion elements, in particular said anti-torsion elements comprising lateral faces of the ball joint (83G) provided with elements tangent to the inner faces of said fork (33).

10. Device (1) according to one or more of the preceding claims, **characterized in that** said device (1) comprises a motor (100) associated with the upper body (82) of said actuation element (80), in particular through the interposition of a reducer (101).

11. Device (1) according to one or more of the preceding claims, **characterized in that** said device (1) comprises:
- a first elastic element (44), which connects the calcaneus element (30) to the medial phalanx element (60) and which is positioned under the medial metatarsal element (40),
- a second elastic element (54), which connects the calcaneus element (30) to the lateral phalanx element (70) and which is positioned under the lateral metatarsal element (50),
in particular said device (1) comprising a first system (45, 46) for adjusting the tension of the first elastic element (44) and a second system (55, 56) for adjusting the tension of the second elastic element (54).

12. Device (1) according to one or more of the preceding claims, **characterized in that** the underside of the medial metatarsal element (40) comprises a first arm (47), whereon the first elastic element (44) rests, and the underside of the lateral metatarsal element (50) comprises a second arm (57), whereon the second elastic element (54) rests.

13. Device (1) according to one or more of claims 11 and 12, **characterized in that** the medial phalanx element (60) comprises a first cam (63), whereon the first elastic element (44) rests, and the lateral phalanx element (70) comprises a second cam (73), whereon the second elastic element (54) rests.

14. Device (1) according to one or more of the preceding claims, **characterized in that** said device (1) comprises a locking element for the third joint (A3) and/or for the fourth joint (A4) and/or for the fifth joint (A5) and/or for the sixth joint (A6).

15. Device (1) according to one or more of the preceding claims, **characterized in that** said device (1) comprises at least one sensor (10E, 10I, 30P, 40P, 50P, 60P, 70P) associated with at least one joint (A1, A2, A3, A4, A5, A6) for providing a direct reading of the data concerning said joint (A1, A2, A3, A4, A5, A6),
in particular said device (1) comprising one or more of the following sensors:
- an encoder (10E), in particular of the magnetic type, fastened to the tibia element (10) at the first joint (A1) for reading the talocrural joint angle, in particular said encoder (10E) being positioned inside a support (10S) fastened to the tibia element (10) by fastening means;
- an inertial sensor (10I) fastened to the tibia element (10) at the first talocrural joint (A1) for reading the absolute tibia angles, in particular said inertial sensor (10I) being housed in the support (10S) of the encoder (10E);
- a first potentiometer (30P) fastened to the calcaneus element (30) at the second joint (A2) for reading the subtalar joint angle;
- a second potentiometer (40P) fastened to the medial metatarsal element (40) at the third joint (A3) for reading the medial midtarsal angle;
- a third potentiometer (50P) fastened to the lateral metatarsal element (50) at the fourth joint (A4) for reading the lateral midtarsal angle;
- a fourth potentiometer (60P) fastened at the fifth joint (A5) for reading the medial metatarsophalangeal angle, in particular said fourth potentiometer (60P) being fastened to the medial metatarsal element (40);
- a fifth potentiometer (70P) fastened at the sixth joint (A6) for reading the lateral metatarsophalangeal angle, in particular said fifth potentiometer (70P) being fastened to the lateral metatarsal element (50).

## Patentansprüche

1. Knöchel-Fuß-Prothese (1), insbesondere vom biomimetischen Typ, wobei die Vorrichtung (1) **dadurch gekennzeichnet ist, dass** sie umfasst:
- ein Tibiaelement (10);
- ein Taluselement (20), das über ein erstes Talokruralgelenk (A1) mit einem ersten Scharniergelenk beweglich mit dem Tibiaelement (10) verbunden ist;
- ein Kalkaneuselement (30), das über ein zweites Subtalargelenk (A2) mit einem zweiten Scharniergelenk beweglich mit dem Taluselement (20) verbunden ist;
- ein mediales Metatarsalelement (40), das mit dem Kalkaneuselement (30) über ein drittes mediales Midtarsalgelenk (A3) beweglich verbunden ist, wobei das dritte Gelenk (A3) ein drittes Scharniergelenk umfasst;
**gekennzeichnet durch**
- ein laterales Metatarsalelement (50), das über ein viertes laterales Midtarsalgelenk (A4) beweglich mit dem Kalkaneuselement (30) verbunden ist, wobei das vierte Gelenk (A4) ein viertes Scharniergelenk umfasst;
- ein mediales Phalanxelement (60), das beweglich mit dem medialen Metatarsalelement (40) über ein fünftes mediales Metatarsophalangealgelenk (A5) verbunden ist, wobei das fünfte Gelenk (A5) ein fünftes Scharniergelenk umfasst;
- ein laterales Phalanxelement (70), das beweglich mit dem lateralen Metatarsalelement (50) durch ein sechstes laterales Metatarsophalangealgelenk (A6) verbunden ist, wobei das sechste Gelenk (A6) ein sechstes Scharniergelenk umfasst;
- ein elastisches Betätigungselement (80), das einen oberen Teil, der beweglich mit dem Tibiaelement (10) verbunden ist, und einen unteren Teil umfasst, der beweglich mit dem Kalkaneuselement (30) verbunden ist.

2. Vorrichtung (1) nach Anspruch 1, **dadurch gekennzeichnet, dass** das Tibiaelement (10) eine gabelförmige Gestalt hat und einen ersten Arm (11) und einen zweiten Arm (12) umfasst, die im Wesentlichen parallel zueinander sind.

3. Vorrichtung (1) gemäß einem oder mehreren der vorstehenden Ansprüche, **dadurch gekennzeichnet, dass** das erste Talokruralgelenk (A1) auf einer ersten Achse (X1) liegt und das zweite Subtalargelenk (A2) auf einer zweiten Achse (X2) liegt, wobei die erste Achse (X1) und die zweite Achse (X2) eine schräge Doppelknöchelachse mit biomimetischer Ausrichtung und Position bilden, wobei insbesondere das erste Talokruralgelenk ( A1) eine erste Öffnung (11A), die mit dem ersten Arm (11) des Tibiaelements (10) verbunden ist, und eine zweite Öffnung (12A), die mit dem zweiten Arm (12) des Tibiaelements (10) verbunden ist, umfasst, wobei die Öffnungen (11A, 12A) mit einem Talokruralstift (21) verbunden sind, der mit dem Taluselement (20) einstückig ist.

4. Vorrichtung (1) gemäß einem oder mehreren der vorstehenden Ansprüche, **dadurch gekennzeichnet, dass** das zweite Subtalargelenk (A2) ein Loch (22) auf dem Taluselement (20) umfasst, das mit einem Subtalarstift (31) verbunden ist, der mit dem Kalkaneuselement (30) einstückig ist.

5. Vorrichtung (1) gemäß einem oder mehreren der vorstehenden Ansprüche, **dadurch gekennzeichnet, dass** das zweite Subtalargelenk (A2) ein Subtalarpolster (23) umfasst, das zwischen gegenüberliegenden Flächen des Taluselements (20) und des Kalkaneuselements (30) angeordnet ist, wobei das Subtalarepolster (23) insbesondere aus einem Elastomermaterial hergestellt ist.

6. Vorrichtung (1) nach einem oder mehreren der vorstehenden Ansprüche, **dadurch gekennzeichnet, dass** das dritte mediale Midtarsalgelenk (A3) und das vierte laterale Midtarsalgelenk (A4) im Wesentlichen parallel und koaxial zueinander sind, wobei diese Gelenke (A3, A4) auf einer dritten Achse (X3) und einer vierten Achse (X4) liegen, die im Wesentlichen zusammenfallen, wobei insbesondere das dritte mediale Midtarsalgelenk (A3) einen ersten Stift (32A) umfasst, der mit dem Kalkaneuselement (30) einstückig ist und mit einem Loch (41) gelenkig verbunden ist, das an einem proximalen Teil des medialen Metatarsalelements (40) vorhanden ist, und insbesondere das vierte laterale Midtarsalgelenk (A4), das einen zweiten Stift (32B) umfasst, der mit dem Kalkaneuselement (30) einstückig ist und mit einem Loch (51) gelenkig verbunden ist, das auf einem proximalen Teil des lateralen Metatarsalelements (50) vorhanden ist.

7. Vorrichtung (1) gemäß einem oder mehreren der vorstehenden Ansprüche, **dadurch gekennzeichnet, dass** das fünfte mediale Metatarsophalangealgelenk (A5) und das sechste laterale Metatarsophalangealgelenk (A6) auf einer fünften Achse (X5) bzw. einer sechsten Achse (X6) liegen, die im Wesentlichen parallel und nicht deckungsgleich sind, insbesondere das fünfte mediale Metatarsophalangealgelenk (A5) aus einem Scharniergelenk besteht, das einen medialen Metatarsophalangealstift (42) umfasst, der mit mindestens einer ersten Öffnung (43), die an einem distalen Teil des medialen Metatarsalelements (40) vorhanden ist, und mit einer zweiten Öffnung (62), die an einem Arm (61) vorhanden ist, der sich vom medialen Phalanxelement (60) erstreckt, gelenkig verbunden ist,
und insbesondere das sechste laterale Metatarsophalangealgelenk (A6) aus einem Scharniergelenk besteht, das einen lateralen Metatarsophalangealstift (52) umfasst, der mit mindestens einer ersten Öffnung (53), die an einem distalen Teil des lateralen Metatarsalelements (50) vorhanden ist, und mit einer zweiten Öffnung (72), die an einem Arm (71) vorhanden ist, der sich von dem lateralen Phalanxelement (70) erstreckt, gelenkig verbunden ist.

8. Vorrichtung (1) gemäß einem oder mehreren der vorstehenden Ansprüche, **dadurch gekennzeichnet, dass** das Betätigungselement (80) eine Feder (81) umfasst, die mit einem oberen Körper (82) verbunden ist, der mit dem Tibiaelement (10) über ein schwenkbares Gabelsystem gelenkig verbunden ist, das zwei Freiheitsgrade zwischen dem Betätigungselement (80) und dem Tibiaelement (10) ermöglicht, wobei das schwenkbare Gabelsystem insbesondere Folgendes umfasst:
- ein gabelförmiges Element (90),
- erste Verbindungselemente, die eine erste relative Drehbewegung zwischen dem Betätigungselement (80) und dem gabelförmigen Element (90) ermöglichen,
- zweite Verbindungselemente (84, 85, 86, 87), die eine zweite relative Drehbewegung zwischen dem Betätigungselement (80) und dem gabelförmigen Element (90) ermöglichen,
insbesondere die ersten Verbindungselemente Radiallager (82C) umfassen, die mit dem oberen Körper (82) des Betätigungselements (80) verbunden sind und mit jeweiligen Stiften (91P) gelenkig verbunden sind, die mit den Armen (91) des gabelförmigen Elements (90) verbunden sind,
und insbesondere die zweiten Verbindungselemente mindestens ein Lager (84, 85, 86) umfassen, das an einer Stange (92) des gabelförmigen Elements (90) angebracht ist, das mit mindestens einem jeweiligen Sitz, der dem Tibiaelement (10) zugeordnet ist, gelenkig verbunden ist.

9. Vorrichtung (1) gemäß einem oder mehreren der vorstehenden Ansprüche, **dadurch gekennzeichnet, dass** der untere Teil des Betätigungselements (80) mit dem Kalkaneuselement (30) über ein Verbindungsstück (83) gelenkig verbunden ist, das zwei Freiheitsgrade zwischen dem Betätigungselement (80) und dem Kalkaneuselement (30) ermöglicht, wobei insbesondere das Verbindungsstück (83) mit einer Gabel (33) des Kalkaneuselements (30) gelenkig verbunden ist und ein Kugelgelenk (83G) umfasst, das mit Anti-Torsionselementen verbunden ist, wobei die Anti-Torsionselemente insbesondere Seitenflächen des Kugelgelenks (83G) umfassen, die mit Elementen versehen sind, die die Innenflächen der Gabel (33) berühren.

10. Vorrichtung (1) gemäß einem oder mehreren der vorstehenden Ansprüche, **dadurch gekennzeichnet, dass** die Vorrichtung (1) einen Motor (100) umfasst, der mit dem oberen Körper (82) des Betätigungselements (80) verbunden ist, insbesondere durch Zwischenschaltung eines Untersetzungsgetriebes (101).

11. Vorrichtung (1) gemäß einem oder mehreren der vorstehenden Ansprüche, **dadurch gekennzeichnet, dass** die Vorrichtung (1) umfasst:
- ein erstes elastisches Element (44), das das Kalkaneuselement (30) mit dem medialen Phalanxelement (60) verbindet und unter dem medialen Metatarsalelement (40) angeordnet ist,
- ein zweites elastisches Element (54), das das Kalkaneuselement (30) mit dem lateraten Phalanxelement (70) verbindet und unter dem lateralen Metatarsalelement (50) angeordnet ist,
insbesondere umfasst die Vorrichtung (1) ein erstes System (45, 46) zum Einstellen der Spannung des ersten elastischen Elements (44) und ein zweites System (55, 56) zum Einstellen der Spannung des zweiten elastischen Elements (54).

12. Vorrichtung (1) gemäß einem oder mehreren der vorstehenden Ansprüche, **dadurch gekennzeichnet, dass** die Unterseite des medialen Metatarsalelements (40) einen ersten Arm (47) umfasst, auf dem das erste elastische Element (44) aufliegt, und die Unterseite des lateralen Metatarsalelements (50) einen zweiten Arm (57) umfasst, auf dem das zweite elastische Element (54) aufliegt.

13. Vorrichtung (1) gemäß einem oder mehreren der Ansprüche 11 und 12, **dadurch gekennzeichnet, dass** das mediale Phalanxelement (60) einen ersten Nocken (63) umfasst, auf dem das erste elastische Element (44) aufliegt, und das laterale Phalanxelement (70) einen zweiten Nocken (73) umfasst, auf dem das zweite elastische Element (54) auf liegt.

14. Vorrichtung (1) gemäß einem oder mehreren der vorstehenden Ansprüche, **dadurch gekennzeichnet, dass** die Vorrichtung (1) ein Verriegelungselement für das dritte Gelenk (A3) und/oder für das vierte Gelenk (A4) und/oder für das fünfte Gelenk (A5) und/oder für das sechste Gelenk (A6) umfasst.

15. Vorrichtung (1) gemäß einem oder mehreren der vorstehenden Ansprüche, **dadurch gekennzeichnet, dass** die Vorrichtung (1) mindestens einen Sensor (10E, 101, 30P, 40P, 50P, 60P, 70P) umfasst, der mit mindestens einem Gelenk (A1, A2, A3, A4, A5, A6) verbunden ist, um eine direkte Ablesung der Daten bezüglich des Gelenks (A1, A2, A3, A4, A5, A6) zu ermöglichen,
wobei insbesondere die Vorrichtung (1) einen oder mehrere der folgenden Sensoren umfasst:
- einen Kodierer (10E), insbesondere vom magnetischen Typ, der an dem Tibiaelement (10) an dem ersten Gelenk (A1) befestigt ist, um den Winkel des Talokruralgelenks zu lesen, wobei insbesondere der Kodierer (10E) innerhalb einer Halterung (10S) positioniert ist, der an dem Tibiaelement (10) durch Befestigungsmittel befestigt ist;
- einen Trägheitssensor (101), der am Tibiaelement (10) am ersten Talokruralgelenk (A1) befestigt ist, um die absoluten Tibiawinkel abzulesen, wobei insbesondere der Trägheitssensor (101) in der Halterung (10S) des Kodierers (10E) untergebracht ist;
- ein erstes Potentiometer (30P), das am Kalkaneuselement (30) am zweiten Gelenk (A2) befestigt ist, um den Winkel des Subtalargelenks abzulesen;
- ein zweites Potentiometer (40P), das am medialen Metatarsalelement (40) am dritten Gelenk (A3) befestigt ist, um den Winkel des medialen Midtarsals abzulesen;
- ein drittes Potentiometer (50P), das am lateralen Metatarsalelement (50) am vierten Gelenk (A4) befestigt ist, um den Winkel des lateralen Midtarsals abzulesen;
- ein viertes Potentiometer (60P), das am fünften Gelenk (A5) befestigt ist, um den Winkel des medialen Metatarsophalangeals abzulesen, wobei insbesondere das vierte Potentiometer (60P) am medialen Metatarsalelement (40) befestigt ist;
- ein fünftes Potentiometer (70P), das am sechsten Gelenk (A6) befestigt ist, um den Winkel des lateralen Metatarsophalangeals abzulesen, wobei insbesondere das fünfte Potentiometer (70P) am lateralen Metatarsalelement (50) befestigt ist.

## Revendications

1. - Dispositif prothétique de cheville-pied (1), en particulier du type biomimétique, ledit dispositif (1) étant **caractérisé par le fait qu'**il comprend :
- un élément tibia (10) ;
- un élément astragale (20) relié de manière mobile à l'élément tibia (10) par l'intermédiaire d'une première articulation talo-crurale (Al) comprenant une première articulation à charnière ;
- un élément calcanéum (30) relié de manière mobile à l'élément astragale (20) par l'intermédiaire d'une deuxième articulation sous-astragalienne (A2) comprenant une deuxième articulation à charnière ;
- un élément métatarsien médian (40) relié de manière mobile à l'élément calcanéum (30) par l'intermédiaire d'une troisième articulation médio-tarsienne médiane (A3), ladite troisième articulation (A3) comprenant une troisième articulation à charnière ;
**caractérisé par** :
- un élément métatarsien latéral (50) relié de manière mobile à l'élément calcanéum (30) par l'intermédiaire d'une quatrième articulation médio-tarsienne latérale (A4), ladite quatrième articulation (A4) comprenant une quatrième articulation à charnière ;
- un élément phalange médian (60) relié de manière mobile à l'élément métatarsien médian (40) par l'intermédiaire d'une cinquième articulation métatarso-phalangienne médiane (A5), ladite cinquième articulation (A5) comprenant une cinquième articulation à charnière ;
- un élément phalange latéral (70) relié de manière mobile à l'élément métatarsien latéral (50) par l'intermédiaire d'une sixième articulation métatarso-phalangienne latérale (A6), ladite sixième articulation (A6) comprenant une sixième articulation à charnière ;
- un élément d'actionnement élastique (80) comprenant une partie supérieure reliée de manière mobile à l'élément tibia (10) et une partie inférieure reliée de manière mobile à l'élément calcanéum (30).

2. - Dispositif (1) selon la revendication 1, **caractérisé par le fait que** l'élément tibia (10) a une forme de type fourche et comprend un premier bras (11) et un second bras (12) sensiblement parallèles l'un à l'autre.

3. - Dispositif (1) selon une ou plusieurs des revendications précédentes, **caractérisé par le fait que** la première articulation talo-crurale (Al) se situe sur un premier axe (XI) et la deuxième articulation sous-astragalienne (A2) se situe sur un deuxième axe (X2), dans lequel ledit premier axe (XI) et ledit deuxième axe (X2) forment un double axe de cheville oblique ayant une orientation et une position biomimétiques, en particulier la première articulation talo-crurale (A1) comprenant une première ouverture (11A) associée au premier bras (11) de l'élément tibia (10) et une seconde ouverture (12A) associée au second bras (12) de l'élément tibia (10), lesdites ouvertures (11 A, 12A) étant couplées à une broche talo-crurale (21) solidaire de l'élément astragale (20).

4. - Dispositif (1) selon une ou plusieurs des revendications précédentes, **caractérisé par le fait que** la deuxième articulation sous-astragalienne (A2) comprend un trou (22) sur l'élément astragale (20), qui est couplé à une broche sous-astragalienne (31) solidaire de l'élément calcanéum (30).

5. - Dispositif (1) selon une ou plusieurs des revendications précédentes, **caractérisé par le fait que** la deuxième articulation sous-astragalienne (A2) comprend un coussinet sous-astragalien (23) positionné entre des faces opposées de l'élément astragale (20) et de l'élément calcanéum (30), en particulier ledit coussinet sous-astragalien (23) étant fait de matériau élastomère.

6. - Dispositif (1) selon une ou plusieurs des revendications précédentes, **caractérisé par le fait que** la troisième articulation médio-tarsienne médiane (A3) et la quatrième articulation médio-tarsienne latérale (A4) sont sensiblement parallèles et coaxiales l'une à l'autre, lesdites articulations (A3, A4) se situant respectivement sur un troisième axe (X3) et un quatrième axe (X4) qui coïncident sensiblement, en particulier la troisième articulation médio-tarsienne médiane (A3) comprenant une première broche (32A) solidaire de l'élément calcanéum (30), qui s'articule avec un trou (41) qui est présent sur une partie proximale de l'élément métatarsien médian (40), et en particulier la quatrième articulation médio-tarsienne latérale (A4) comprenant une deuxième broche (32B) solidaire de l'élément calcanéum (30), qui s'articule avec un trou (51) qui est présent sur une partie proximale de l'élément métatarsien latéral (50).

7. - Dispositif (1) selon une ou plusieurs des revendications précédentes, **caractérisé par le fait que** la cinquième articulation métatarso-phalangienne médiane (A5) et la sixième articulation métatarso-phalangienne latérale (A6) se situent sur un cinquième axe (X5) et un sixième axe (X6), respectivement, qui sont sensiblement parallèles et non coïncidents,
en particulier la cinquième articulation métatarso-phalangienne médiane (A5) étant constituée d'une articulation à charnière comprenant une broche métatarso-phalangienne médiane (42) qui s'articule avec au moins une première ouverture (43) qui est présente sur une partie distale de l'élément métatarsien médian (40) et avec une seconde ouverture (62) qui est présente sur un bras (61) s'étendant à partir de l'élément phalange médian (60), et en particulier la sixième articulation métatarso-phalangienne latérale (A6) étant constituée d'une articulation à charnière comprenant une broche métatarso-phalangienne latérale (52) qui s'articule avec au moins une première ouverture (53) qui est présente sur une partie distale de l'élément métatarsien latéral (50) et avec une seconde ouverture (72) qui est présente sur un bras (71) s'étendant à partir de l'élément phalange latéral (70).

8. - Dispositif (1) selon une ou plusieurs des revendications précédentes, **caractérisé par le fait que** l'élément d'actionnement (80) comprend un ressort (81) relié à un corps supérieur (82) qui s'articule avec l'élément tibia (10) par l'intermédiaire d'un système de fourche pivotante autorisant deux degrés de liberté entre ledit élément d'actionnement (80) et l'élément tibia (10), en particulier ledit système de fourche pivotante comprenant :
- un élément en forme de fourche (90),
- des premiers éléments de liaison qui autorisent un premier mouvement de rotation relatif entre l'élément d'actionnement (80) et l'élément en forme de fourche (90),
- des seconds éléments de liaison (84, 85, 86, 87) qui autorisent un second mouvement de rotation relatif entre l'élément d'actionnement (80) et l'élément en forme de fourche (90),
en particulier lesdits premiers éléments de liaison comprenant des roulements radiaux (82C) associés au corps supérieur (82) de l'élément d'actionnement (80), qui s'articulent avec des broches (91P) respectives associées aux bras (91) de l'élément en forme de fourche (90), et
en particulier lesdits seconds éléments de liaison comprenant au moins un roulement (84, 85, 86) monté sur une tige (92) de l'élément en forme de fourche (90), qui s'articule avec au moins un siège respectif associé à l'élément tibia (10).

9. - Dispositif (1) selon une ou plusieurs des revendications précédentes, **caractérisé par le fait que** la partie inférieure de l'élément d'actionnement (80) s'articule avec l'élément calcanéum (30) par l'intermédiaire d'une extrémité (83) qui autorise deux degrés de liberté entre ledit élément d'actionnement (80) et l'élément calcanéum (30), en particulier ladite extrémité (83) s'articulant avec une fourche (33) de l'élément calcanéum (30) et comprenant une rotule (83G) associée à des éléments anti-torsion, en particulier lesdits éléments anti-torsion comprenant des faces latérales de la rotule (83G) comportant des éléments tangents aux faces internes de ladite fourche (33).

10. - Dispositif (1) selon une ou plusieurs des revendications précédentes, **caractérisé par le fait que** ledit dispositif (1) comprend un moteur (100) associé au corps supérieur (82) dudit élément d'actionnement (80), en particulier par l'interposition d'un réducteur (101).

11. - Dispositif (1) selon une ou plusieurs des revendications précédentes, **caractérisé par le fait que** ledit dispositif (1) comprend :
- un premier élément élastique (44), qui relie l'élément calcanéum (30) à l'élément phalange médian (60) et qui est positionné sous l'élément métatarsien médian (40),
- un second élément élastique (54), qui relie l'élément calcanéum (30) à l'élément phalange latéral (70) et qui est positionné sous l'élément métatarsien latéral (50), en particulier ledit dispositif (1) comprenant un premier système (45, 46) pour régler la tension du premier élément élastique (44) et un second système (55, 56) pour régler la tension du second élément élastique (54).

12. - Dispositif (1) selon une ou plusieurs des revendications précédentes, **caractérisé par le fait que** le côté inférieur de l'élément métatarsien médian (40) comprend un premier bras (47), sur lequel repose le premier élément élastique (44), et le côté inférieur de l'élément métatarsien latéral (50) comprend un second bras (57), sur lequel repose le second élément élastique (54).

13. - Dispositif (1) selon une ou plusieurs des revendications 11 et 12, **caractérisé par le fait que** l'élément phalange médian (60) comprend une première came (63), sur laquelle repose le premier élément élastique (44), et l'élément phalange latéral (70) comprend une seconde came (73), sur laquelle repose le second élément élastique (54).

14. - Dispositif (1) selon une ou plusieurs des revendications précédentes, **caractérisé par le fait que** ledit dispositif (1) comprend un élément de verrouillage pour la troisième articulation (A3) et/ou pour la quatrième articulation (A4) et/ou pour la cinquième articulation (A5) et/ou pour la sixième articulation (A6).

15. - Dispositif (1) selon une ou plusieurs des revendications précédentes, **caractérisé par le fait que** ledit dispositif (1) comprend au moins un capteur (10E, 101, 30P, 40P, 50P, 60P, 70P) associé à au moins une articulation (Al, A2, A3, A4, A5, A6) pour fournir une mesure directe des données concernant ladite articulation (Al, A2, A3, A4, A5, A6),
en particulier ledit dispositif (1) comprenant un ou plusieurs des capteurs suivants :
- un codeur (10E), en particulier du type magnétique, fixé à l'élément tibia (10) à la première articulation (Al) pour mesurer l'angle d'articulation talo-crurale, en particulier ledit codeur (10E) étant positionné à l'intérieur d'un support (10S) fixé à l'élément tibia (10) par des moyens de fixation ;
- un capteur inertiel (101) fixé à l'élément tibia (10) à la première articulation talo-crurale (A1) pour mesurer les angles de tibia absolus, en particulier ledit capteur inertiel (101) étant reçu dans le support (10S) du codeur (10E) ;
- un premier potentiomètre (30P) fixé à l'élément calcanéum (30) à la deuxième articulation (A2) pour mesurer l'angle d'articulation sous-astragalienne ;
- un deuxième potentiomètre (40P) fixé à l'élément métatarsien médian (40) à la troisième articulation (A3) pour mesurer l'angle médio-tarsien médian ;
- un troisième potentiomètre (50P) fixé à l'élément métatarsien latéral (50) à la quatrième articulation (A4) pour mesurer l'angle médio-tarsien latéral ;
- un quatrième potentiomètre (60P) fixé à la cinquième articulation (A5) pour mesurer l'angle métatarsophalangien médian, en particulier ledit quatrième potentiomètre (60P) étant fixé à l'élément métatarsien médian (40) ;
- un cinquième potentiomètre (70P) fixé à la sixième articulation (A6) pour mesurer l'angle métatarsophalangien latéral, en particulier ledit cinquième potentiomètre (70P) étant fixé à l'élément métatarsien latéral (50).
